# EUROPÄISCHE PATENTSCHRIFT

(11) **EP 0 610 655 B1**
(45) Veröffentlichungstag und Bekanntmachung des Hinweises auf die Patenterteilung: **12.06.1996**
(21) Anmeldenummer: 94100146.3
(22) Anmeldetag: 07.01.1994
(51) Int. Cl.: A61K 31/16, A61K 47/32

(54) **Arzneimittel mit einem Gehalt an Dexpanthenol zur topischen Anwendung**
Medicament containing dexpanthenol for topical application
Medicament contenant du dexpanthénole pour l'application topique

(30) Priorität: 10.02.1993 DE 4303818
(43) Veröffentlichungstag der Anmeldung: 17.08.1994
(73) Patentinhaber: Dr. GERHARD MANN chem.-pharm. Fabrik GmbH, D-13581 Berlin (DE)
(72) Erfinder: Bellmann, Günther, Dr., 13593 Berlin (DE)
(74) Vertreter: Maiwald, Walter, Dr. Dipl.-Chem.

(56) Entgegenhaltungen:
- WO-A-92/19217
- WO-A-92/19218
- WO-A-92/19275
- DEUTSCHE APOTHEKER ZEITUNG Bd. 130, Nr. 8 , 1990 Seiten 401 - 403 K.-D. BREMECKER ET AL. 'Polyacrylatgele'

## Beschreibung

Die Erfindung betrifft Arzneimittel mit einem Gehalt an Dexpanthenol zur topischen Anwendung.

Pantothensäure ist (R)-3-(2,4-dihydroxy-3,3-dimethylbutyramido)-propyonsäure und kommt in der Natur weitverbreitet in tierischen und pflanzlichem Material vor. Nur die natürliche R-Form der Pantothensäure ist biologisch wirksam, aber auch der der Pantothensäure entsprechende Alkohol, daß Dexpanthenol, zeigt volle Vitaminwirksamkeit, da der Alkohol im Warmblüterorganismus zu Pantothensäure aufoxidiert werden kann. Da Pantothensäure selbst ein unstabiles, visköses stark hygroskopisches Öl ist, ist die technologische Verarbeitung schwierig, so daß pharmazeutisch wegen der besseren Verarbeitungsmöglichkeiten entweder Alkohol oder Salze eingesetzt werden. Pantothensäure und Panthenol sind relativ empfindliche Verbindungen, die durch Säure, Basen und Hitze zersetzt werden, so daß ihre Verarbeitung besondere Maßnahmen erfordert.

Pantothensäure liegt im Gewebe zum überwiegenden Teil als Koenzym A vor, dem eine Schlüsselstellung im Stoffwechsel zukommt. Die Übertragung von Azetylresten, die grundsätzliche Bedeutung im Stoffwechsel besitzt, ist nur mit Hilfe des Koenzym A möglich. Therapeutisch wird Pantothensäure bzw. Panthenol bei Wachstumsstörungen, Dermatitiden, degenerativen Veränderungen des zentralen und peripheren Nervensystems, bei Verminderung der Antikörperbildung und zur Förderung der Wundheilung gegeben.

Bisher üblich ist die Verabreichung von Dexpanthenol in Form von Tabletten, Ampullen, Lösungen oder Salben, da die hohe Instabilität des Dexpanthenol die Herstellung bestimmter pharmazeutischer Zubereitungsformen erschwert.

Für topische Anwendungszwecke wird in der Regel Dexpanthenol in Salbenform gegeben, und zwar auch in der Ophthalmologie. In diesem Bereich, aber auch bei der Behandlung von Hautdefekten, läßt sich häufig feststellen, daß die Patientencompliance bei Salben nur relativ gering ist, da viele Menschen die fetthaltigen Salbengrundlagen auf der Haut oder Hornhaut als unangenehm empfinden. Dies trifft besonders bei Ophthalmologika zu, da fetthaltige Salben mit Tränenflüssigkeit zur Schlierenbildung auf der Hornhaut und damit zu einer Beeinträchtigung des Sehens führen. Dexpanthenolhaltige Gele auf Polyacrylatbasis werden beschrieben von WO-A-92 19218, WO-A-92 19275, WO-A-92 19217 und Deutsche Apothekerzeitung, Band 130, Nr. 8, 1990.

Es besteht daher noch ein Bedürfnis nach pharmazeutischen Zubereitungen mit Dexpanthenol, die topisch anwendbar sind, aber auf die Verwendung fetthaltiger Salbengrundlagen verzichten.

Erfindungsgemäß werden nunmehr sterile Arzneimittel mit einem Gehalt an Dexpanthenol zur topischen Anwendung vorgeschlagen, die dadurch gekennzeichnet sind, daß sie als Träger Polyacrylsäure in teilweise neutralisierter Form mit einem Molekulargewicht zwischen etwa 3 - 5 Mio enthalten.

Die Herstellung von sterilen Arzneimitteln mit einem Gehalt an Dexpanthenol zur topischen Anwendung auf Nichtfettbasis war bisher nicht erfolgreich, da einerseits Dexpanthanol hitzeinstabil ist und in wäßriger Lösung das Zersetzungsprodukt 3 Aminopropanol bildet. Andererseits können aber Polyacrylatgele, wie bekannt, nicht steril filtriert werden. Überraschenderweise hat sich jetzt herausgestellt, daß diese Schwierigkeiten bewältigt werden können, wenn die Herstellung solcher Gele mehrstufig erfolgt. Dazu wird zunächst eine Polyacrylsäuresuspension aus Wasser für Injektionszwecke und einer Polyacrylsäure mit einem Molekulargewicht zwischen etwa 3 - 5 Mio, und vorzugsweise 4 Mio hergestellt und diese Suspension unter Rühren bei etwa 120°C, 1 bar Überdruck und einer Zeitdauer von 20 Minuten autoklaviert.

Parallel dazu wird eine Lösung aus Dexpanthenol, ggf. einem Konservierungsmittel und einem Gelatisierungsmittel hergestellt und in an sich bekannter Weise steril filtriert. Diese Lösung wird unter aseptischen Bedinungen der Polyacrylsäuresuspension zugesetzt. Nach sorgfältigem Vermischen wird die Masse dann mit steriler Natronlauge neutralisiert, wobei sich das Gel bildet. Die Neutralisation ist ein kritischer Punkt bei der Herstellung, da Dexpanthenol säuren- und baseninkompatibel ist, so daß die Zugabe des Neutralisierungsmittels unter Rühren in stark verdünnter Form und unter sofortigem Vermischen erfolgen muß, um eine Zersetzung zu verhindern. Das sterile Gel wird abschliessend durch Evakuieren blasenfrei gemacht und dann in üblicher Weise konfektioniert. Vorzugsweise erfolgt der ganze Herstellungsvorgang unter einem sterilen Inertgas, vorzugsweise Stickstoff.

Als Träger werden Polyacrylsäuren in teilweise neutralisierter Form eingesetzt, wobei die Säuren ein Molekulargewicht im Bereich von etwa 3 - 5 Mio, vorzugsweise 4 Mio aufweisen sollten. Derartige Polyacrylsäuren sind beispielsweise unter der Handelsmarke "Carbopol" im Handel erhältlich. Die Neutralisation der Polyacrylsäuren erfolgt im abschliessenden Herstellungsschritt vorzugsweise mit stark verdünnter steriler Natronlauge, aber anstelle von Natronlauge als Alkali können auch Kaliumhydroxyd, Alkalicarbonate oder Amine wie beispielsweise Triethanolamin oder Diisopropanolamin eingesetzt werden. Ob die Neutralisation mit anorganischen oder organischen Basen erfolgt, ist, solange diese physiologisch unbedenklich sind, nicht von entscheidender Bedeutung.

Vorzugsweise enthalten die erfindungsgemäßen Zubereitungen ein Konservierungsmittel, um auch Keimfreiheit oder Keimarmut nach mehrmaligem Gebrauch zu ermöglichen. Als Konservierungsmittel können die bekannten pharmazeutisch einsetzbaren Verbindungen Anwendung finden, und zwar insbesondere Quats wie beispielsweise Cetrimide. Zum Abfangen von unerwünschenten Metallspuren wird dem Mittel ein Chelatisierungsmittel zugesetzt, wie beispielsweise das Dinatriumsalz der EDTA, Natriumsalze von Phosphonsäuren oder solche der Nitrilotriessigsäure. Völlig überraschend und noch ungeklärt ist der Effekt, daß ein geringer Zusatz von EDTA die Stabilität von Dexpanthenollösungen deutlich erhöht, wobei dieser Effekt, wie verschiedene Untersuchungen gezeigt haben, nicht auf der Chelatisierung von den Spuren möglicherweise vorliegenden Metallionen 2- oder höherwertiger Metalle beruht, sondern über einen anderen Wirkungsmechanismus einzutreten scheint. Jedenfalls haben Untersuchungen hinsichtlich der Lagerstabilität und auch der thermischen Belastbarkeit ergeben, daß ein geringer Zusatz von EDTA eine Zersetzung zu 3-Aminopropanol im Gegensatz zu anderen Komplexbildnern deutlich hemmt.

Die pH-Wert-Einstellung des fertigen Gels erfolgt auf pH 6,8 plus minus 0,2, so daß die fertigen Gele dem physiologischem pH entsprechen. Daher werden diese Gele bei der Verwendung am Auge oder auf Schleimhäuten genauso reaktionslos vertragen wie bei der Anwendung auf der Haut, wenn z.B. zur Versorgung großlächiger Hautläsionen wie Brandwunden eine sterile Abdeckung notwendig ist.

Die Erfindung wird im folgenden anhand eines Beispieles näher erläutert:

### Beispiel

In einer Becomix-Prozeßanlage mit Sterilfilter 0,2 µm und einem Gassterilfilter wird eine homogene Suspension über einen Faserfangfilter mit einer Porenweite von etwa 25 - 40 µm aus 160,0 kg Wasser für Injektionszwecke und 1,14 kg Polyacrylsäure MG 4 Mio (im Handel erhältlich unter der Marke "Carbopol 940") eingebracht. Danach wird die Anlage geschlossen und aufgeheizt. Die Suspension wird unter Rühren bei 121°C und 1 bar Überdruck 20 Minuten autoklaviert und anschliessend auf Raumtemperatur heruntergekühlt, wobei über das sterilisierte Luftfilter belüftet wird.

Zwischenzeitlich werden in einem geeigneten Ansatzbehälter 180,0 kg Wasser für Injektionszwecke mit einer Temperatur von ca. 40°C vorgelegt und unter Rühren 19,0 kg Dexpanthenol darin gelöst. Ein Teil dieser Lösung, ca. 10 l, wird abgenommen und in dieser Teilmenge werden unter Rühren 38 g Cetrimide und 38 g des Nitratriumsalzes der EDTA · 2H₂O gelöst. Diese Teillösung wird wieder mit der Hauptmenge der Dexpanthenollösung vereint.

Die Salzlösung wird dann mit Hilfe von sterilem Stickstoff als Druckgas über das Sterilfilter zur bereits autoklavierten Polyacrylsäuresuspension hinzugefügt. Die Mischung wird anschliessend durch Rühren homogenisiert. Abschliessend wird mehrfach Evakuiert, und zwar bis 0,1 bar und über das Luftfilter wieder belüftet, um den entstandenen Schaum zu zerstören.

Unter sterilen Bedindungen werden in 19,203 kg Wasser für Injektionszwecke 581 g Natriumhydroxyd unter Rühren gelöst, so daß eine etwa 3%-ige Natriumhydroxydlösung entsteht. Diese Natronlauge wird dann über das Sterilfilter in die Dexpanthenol-Polyacrylsäuresuspension filtriert, wobei gleichzeitig gerührt und das sich bildende Gel umgewälzt wird. Die Zuführung der Natronlauge erfolgt tropfenweise.

Vom entstandenen Gel wird der pH-Wert bestimmt, der bei 6,8 plus minus 0,2 liegen soll. Liegt der pH-Wert unterhalb 6,6 wird vorsichtig tropfenweise solange Natriumhydroxidlösung zugegeben, bis der gewünschte Wert erreicht ist. Dabei ist zu berücksichtigen, daß zwar bei der Alkalizugabe regelmäßig gerührt werden muß, um eine Zersetzung des Dexpanthenol zu verhindern, daß aber andererseits zu langes Rühren bei höherer Geschwindigkeit die Gelstruktur zerstören kann. Wenn bereits für die Mischung ein pH über 6,6 gemessen wird, wird zu einem Gesamtgewicht von 380,00 kg mit sterilem Wasser aufgefüllt.

Die Masse wird dann noch einmal ca. 15 Minuten bei geringer Rührwerksdrehzahl von ca. 8 Upm gerührt und abschlissend solange Evakuiert, bis das Gel praktisch blasenfrei ist. Das fertige Gel wird dann in an sich bekannter Weise unter sterilen Bedingungen konfektioniert.

## Patentansprüche

1. Sterile Arzneimittel mit einem Gehalt an Dexpanthenol zur topischen Anwendung, dadurch gekennzeichnet, daß sie als Träger Polyacrylsäure in teilweise neutralisierter Form mit einem Molekulargewicht zwischen etwa 3 - 5 Mio enthalten.

2. Sterile Arzneimittel nach Anspruch 1, dadurch gekennzeichnet, daß die Polyacrylsäure mit einem Molekulargewicht von 4 Mio enthalten.

3. Arzneimittel nach Anspruch 1 oder 2, dadurch gekennzeichnet, daß die Polyacrylsäure als Alkali, insbesondere Natrium- oder Aminsalz vorliegt.

4. Arzneimittel nach Anspruch 1 bis 3, dadurch gekennzeichnet, daß der pH-Wert des Polyacrylsäuregeles 6,8 plus minus 0,2 beträgt.

5. Arzneimittel nach Anspruch 1 bis 4, dadurch gekennzeichnet, daß das Polyacrylsäuregel ein Konservierungsmittel enthält.

6. Arzneimittel nach Anspruch 1 bis 5, dadurch gekennzeichnet, daß das Gel ein Chelatisierungsmittel enthält.

7. Arzneimittel nach Anspruch 1 bis 6, dadurch gekennzeichnet, daß das Gel EDTA enthält.

8. Verfahren zur Herstellung von sterilen Arzneimitteln nach Anspruch 1, dadurch gekennzeichnet, daß eine Polyacrylsäurelösung autoklaviert, eine Lösung mit Dexpanthenol sowie ggf. Konservierungs- und Chelatisierungsmittel steril filtriert, beide Lösungen vereint und die Mischung mit der vorgesehenen sterilen Base auf pH 6,8 plus minus 0,2 eingestellt wird.

9. Verfahren nach Anspruch 8, dadurch gekennzeichnet, daß die neutralisierte Mischung durch Evakuieren blasenfrei gemacht wird.

10. Verfahren nach Anspruch 8, dadurch gekennzeichnet, daß im gesamten Verfahrensablauf unter sterilem Inertgas gearbeitet wird.

## Claims

1. Sterile pharmaceuticals with a content of dexpanthenol for topical use,
**characterized in** that they contain as vehicle polyacrylic acid in partially neutralized form with a molcular weight between about 3 - 5 million.

2. Sterile pharmaceuticals according to Claim 1,
**characterized in** that they contain polyacrylic acid with a molecular weight of 4 million.

3. Pharmaceuticals according to Claim 1 or 2,
**characterized in** that the polyacrylic acid is in the form of alkali metal, in particular sodium, or amine salt.

4. Pharmaceuticals according to Claim 1 to 3,
**characterized in** that the pH of the polyacrylic acid gel is 6.8 plus minus 0.2.

5. Pharmaceuticals according to Claim 1 to 4,
**characterized in** that the polyacrylic acid gel contains a preservative.

6. Pharmaceuticals according to Claim 1 to 5,
**characterized in** that the gel contains a chelating agent.

7. Pharmaceuticals according to Claim 1 to 6,
**characterized in** that the gel contains EDTA.

8. Process for the production of sterile pharmaceuticals according to Claim 1,
**characterized in** that a polyacrylic acid solution is autoclaved, a solution containing dexpanthenol and, where appropriate, preservatives and chelating agents is sterilized by filtration, the two solutions are combined, and the mixture is adjusted with the intended sterile base to pH 6.8 plus minus 0.2.

9. Process according to Claim 8,
**characterized in** that the neutralized mixture is rendered free of bubbles by evacuation.

10. Process according to Claim 8,
**characterized in** that the entire process is carried out under sterile inert gas.

## Revendications

1. Médicaments stériles contenant du dexpanthénol pour application locale, caractérisés en ce qu'ils contiennent, en tant que véhicule, du poly(acide acrylique) sous forme partiellement neutralisée, ayant une masse moléculaire comprise entre environ 3 millions et 5 millions.

2. Médicaments stériles selon la revendication 1, caractérisés en ce qu'ils contiennent du poly(acide acrylique) ayant une masse moléculaire de 4 millions.

3. Médicaments selon la revendication 1 ou 2, caractérisés en ce que le poly(acide acrylique) est présent sous forme de sels alcalins, en particulier de sels de sodium ou d'amine.

4. Médicaments selon l'une des revendications 1 à 3, caractérisés en ce que le pH du gel de poly(acide acrylique) est de 6,8 ± 0,2.

5. Médicaments selon l'une des revendications 1 à 4, caractérisés en ce que le gel de poly(acide acylique) contient un conservateur.

6. Médicaments selon l'une des revendications 1 à 5, caractérisés en ce que le gel contient un agent chélateur.

7. Médicaments selon l'une des revendications 1 à 6, caractérisés en ce que le gel contient de l'EDTA.

8. Procédé pour la fabrication de médicaments stériles selon la revendication 1, caractérisé en ce que l'on soumet à un autoclavage une solution de poly(acide acrylique), on stérilise par filtration une solution contenant du dexpanthénol ainsi qu'éventuellement un conservateur et un agent chélateur, on réunit les deux solutions et on ajuste à pH 6,8 ± 0,2 le mélange, à l'aide de la base stérile prévue.

9. Procédé selon la revendication 8, caractérisé en ce que le mélange neutralisé est rendu exempt de bulles par mise sous vide.

10. Procédé selon la revendication 8, caractérisé en ce que, dans tout le déroulement du procédé, on opère sous un gaz inerte stérile.
